# EUROPÄISCHE PATENTSCHRIFT

(11) **EP 1 649 820 B1**
(45) Veröffentlichungstag und Bekanntmachung des Hinweises auf die Patenterteilung: **05.09.2007**
(21) Anmeldenummer: 06000663.2
(22) Anmeldetag: 27.05.2002
(51) Int. Cl.: A61B 18/12

(54) **Elektrochirurgische Vorrichtung**
Electrosurgical apparatus
Appareil électrochirurgical

(30) Priorität: 08.06.2001 DE 10128377
(43) Veröffentlichungstag der Anmeldung: 26.04.2006
(62) Teilanmeldung aus: 02753060.9
(73) Patentinhaber: Storz-Endoskop GmbH, 8200 Schaffhausen (CH)
(72) Erfinder: Novak, Pavel, 8234 Stetten (CH)
(74) Vertreter: Heuckeroth, Volker

(56) Entgegenhaltungen:
- EP-A- 0 186 369
- DE-A- 2 429 021
- DE-A1- 3 045 996

## Beschreibung

Die Erfindung betrifft eine elektrochirurgische Vorrichtung nach dem Anspruch 1.

Eine elektrochirurgische Vorrichtung nach dem Oberbegriff des Anspruchs 1 ist aus dem Dokument DE 24 29 021 A bekannt.

Eine Vorrichtung der eingangs genannten Art wird in der sogenannten Elektrochirurgie verwendet. Von dem HF-Generator erzeugte Hochfrequenzströme werden über das mit dem HF-Generator verbundene HF-Instrument insbesondere im Rahmen der minimal-invasiven Chirurgie in den Körper eines Patienten geleitet, um an einer Operationsstelle mit dem HF-Instrument unter Wirkung der Hochfrequenzströme Gewebe zu koagulieren und/oder zu schneiden. Beim Koagulieren werden Gefäße verödet, um eine Blutstillung beim Abtrennen von Gewebe herbeizuführen. Das Koagulieren und das Schneiden mittels Hochfrequenzstrom unterscheiden sich hinsichtlich der applizierten Leistung des Hochfrequenzstromes und gegebenenfalls hinsichtlich der Dauer der Applikation. Im Koagulationsmodus einer elektrochirurgischen Vorrichtung wird in der Regel mit kleineren HF-Leistungen und intermittierend gearbeitet, während zum Schneiden von Gewebe mittels Hochfrequenzstrom höhere HF-Leistungen erforderlich sind, um den zum Schneiden von Gewebe mittels Hochfrequenzstrom erforderlichen Lichtbogen zu erzeugen. Des weiteren wird beim Schneiden auch nicht intermittierend, sondern kontinuierlich gearbeitet.

Entsprechend diesen beiden zuvor beschriebenen Betriebsarten des Koagulierens und Schneidens ist der HF-Generator einer derartigen elektrochirurgischen Vorrichtung in der Lage, Hochfrequenzströme mit den entsprechenden für das Koagulieren und Schneiden erforderlichen Leistungen bereitzustellen.

Beim Arbeiten mit einem HF-Instrument werden jedoch in der Regel die Betriebsarten "Koagulieren" und "Schneiden" nicht simultan, sondern abwechselnd benötigt, d.h. die Arbeitsschritte des Schneidens und des Koagulierens finden nacheinander und abwechselnd, jedoch nicht gleichzeitig statt. Der HF-Generator kann entsprechend mit den Betriebsarten "Koagulieren" und "Schneiden" aktiviert werden.

Damit der das HF-Instrument bedienende Chirurg die Aktivierung der Betriebsarten nicht am abseits des Operationstisches und im nicht-sterilen Bereich des Operationssaales stehenden HF-Generator vornehmen, oder entsprechend eine Assistenzperson zum Umschalten zwischen den Betriebsarten anweisen muß, ist bei dem HF-Instrument selbst eine Aktivierungsmöglichkeit der Betriebsarten in Form von zwei mit den Fingern bedienbaren Schaltern vorgesehen.

Dem ersten Schalter ist beispielsweise die Betriebsart "Koagulieren" und dem zweiten Schalter die Betriebsart "Schneiden" zugeordnet. Dem ersten Schalter und dem zweiten Schalter entsprechen somit jeweils ein erster Betriebszustand, beispielsweise höhere Ausgangsleistung des HF-Generators, bzw. ein zweiter Betriebszustand, beispielsweise niedrigere Ausgangsleistung mit einem gegebenenfalls intermittierenden Betrieb.

Der erste Schalter und der zweite Schalter sind mit zumindest einer Steuersignalleitung verbunden, die mit der Hauptleitung des HF-Instruments, über die die aktive Elektrode am distalen Ende des HF-Instruments gespeist wird, verbunden ist. Über die Hauptleitung wird zusätzlich zu dem Hochfrequenzstrom ein Steuereingangssignal eingespeist, aus dem, je nachdem, welcher der beiden Schalter betätigt wird, mittels der Signalcodiermittel ein entsprechendes Steuerausgangssignal zum Aktivieren des dem betätigten Schalter zugeordneten Betriebszustandes erzeugt wird, das über diejenige Steuersignalleitung, mit der der jeweilige Schalter verbunden ist, dem HF-Generator zugespeist wird, um den entsprechenden Betriebszustand des HF-Generators zu aktivieren.

Die aus dem oben genannten Dokument DE 24 29 021 A bekannte elektrochirurgische Vorrichtung weist ein HF-Instrument auf, das drei Schalter aufweist. Wird der erste Schalter geschlossen, so fließt eine Halbwelle des Wechselstroms, um ein erstes Relais zu erregen. Wird der zweite Schalter geschlossen, so fließt die andere Halbwelle des Wechselstroms, um ein zweites Relais zu erregen. Beim Schließen des dritten Schalters wird die Hauptleitung mit der Signalleitung unmittelbar kurzgeschlossen, so daß beide Halbwellen des Wechselstroms fließen können.

Bei einer aus der DE 30 45 996 A1 bekannten Vorrichtung sind an dem HF-Instrument neben den zuvor erwähnten beiden Schaltern zum Umschalten zwischen den Betriebsarten "Koagulieren" und "Schneiden" noch weitere Schalter vorgesehen, nämlich zum jeweiligen Erhöhen bzw. Erniedrigen der Ausgangsleistung in der jeweils gewählten Betriebsart. Bei dieser bekannten Vorrichtung werden zusätzliche Signalleitungen benötigt, um die weiteren Funktionen zum Umschalten des HF-Generators zu realisieren.

Eine damit vergleichbare Vorrichtung ist aus der EP 0 186 369 A1 bekannt, die ebenfalls vier Schalter zum Umschalten zwischen den Betriebsarten "Koagulieren" und "Schneiden" sowie zum Erhöhen bzw. Erniedrigen der Ausgangsleistung in jeder der beiden Betriebsarten aufweist. Bei dieser bekannten Vorrichtung werden insgesamt für die vier Schalter drei Signalleitungen benötigt.

Der Erfindung liegt daher die Aufgabe zugrunde, eine elektrochirurgische Vorrichtung der eingangs genannten Art dahingehend weiterzubilden, daß mit möglichst geringem konstruktivem Aufwand eine Aktivierungsmöglichkeit des ersten, zweiten und zumindest eines drittes Betriebszustands des HF-Generators vom HF-Instrument aus geschaffen wird.

Erfindungsgemäß wird diese Aufgabe hinsichtlich der eingangs genannten elektrochirurgischen Vorrichtung dadurch gelöst, daß dem dritten Schalter eigene Signalcodiermittel zugeordnet sind.

Anstatt bei mehr als zwei vorgesehenen Schaltern zusätzliche Steuersignalleitungen im HF-Instrument vorzusehen, ist bei der erfindungsgemäßen Vorrichtung der konstruktive Aufwand bei der Realisierung des Umschaltens zu einer dritten Betriebsart des HF-Generators dadurch vorteilhaft geringgehalten, daß auch der dritte Schalter mit der jeweiligen vorhandenen, beispielsweise gemeinsamen, Steuersignalleitung verbunden ist, wodurch zusätzliche Steuersignalleitungen eingespart werden. Die Signalcodiermittel zum Erzeugen der Steuerausgangssignale sind vorzugsweise so ausgebildet, daß bei Betätigung des dritten Schalters aus dem Steuereingangssignal, das vorzugsweise für alle drei Schalter das gleiche ist, ein zusätzliches Steuerausgangssignal erzeugt wird, das sich von den Steuerausgangssignalen zum Aktivieren des ersten und zweiten Betriebszustandes unterscheidet, um den dritten Betriebszustand des HF-Generators zu aktivieren.

Dieses weitere Steuerausgangssignal wird über die gemeinsame Steuersignalleitung dem HF-Generator zugespeist.

Erfindungsgemäß sind dem dritten Schalter eigene Signalcodiermittel zugeordnet sind.

Diese Maßnahme hat insbesondere im Zusammenhang mit der Ausgestaltung den Vorteil, daß die zumindest dritte Schaltfunktion eine Kombination der ersten und zweiten Schaltfunktion darstellt, weil eine bessere "Gleichzeitigkeit" der Betätigung der zumindest dritten Schaltfunktion erreicht wird, als wenn zur Realisierung der dritten Schaltfunktion die erste und zweite Schaltfunktion mechanisch miteinander gekoppelt werden.

Dabei ist es weiterhin bevorzugt, wenn die dem dritten Schalter zugeordneten Signalcodiermittel einer Parallelschaltung der dem ersten und zweiten Schalter zugeordneten Signalcodiermittel entsprechen.

Durch diese Maßnahme wird wieder wie bei einer der zuvor genannten Ausgestaltungen eine dritte Schaltfunktion ermöglicht, die zu einem Betriebszustand des HF-Generators führt, der einer Kombination des ersten und zweiten Betriebszustands des HF-Generators entspricht, insbesondere wenn die Signalcodiermittel entsprechende Dioden aufweisen.

Als dritte Betriebsart neben den Betriebsarten "Koagulieren" und "Schneiden" kann bei dieser Ausgestaltung ohne eine spezielle Signalerkennung für den dritten Betriebszustand beispielsweise ein schnelles, abwechselndes Aktivieren der Ausgangsleistung für das Schneiden und der Ausgangsleistung für die Koagulation realisiert werden, wie es in der Artroskopie von Nutzen ist. Andere Betriebszustände können jedoch ebenso in Betracht gezogen werden.

In einer weiteren, ebenso bevorzugten alternativen Ausgestaltung weisen die dem dritten Schalter zugeordneten Signalcodiermittel andere Codiereigenschaften auf als die dem ersten und/oder zweiten Schalter zugeordneten Signalcodiermittel.

Hierbei wird der Vorteil erzielt, daß ohne zusätzliche Steuerleitung eine zusätzliche Codierung erzielt werden kann, die nicht wie zuvor einem Abwechseln des ersten und zweiten Betriebszustandes entspricht, sondern es kann ein gänzlich unterschiedlicher dritter Betriebszustand eingestellt werden. Diese Ausgestaltung eignet sich auch für noch weitere Schalter, bspw. einen vierten oder fünften Schalter. Dabei ist es bevorzugt, wenn die dem dritten Schalter zugeordneten Signalcodiermittel und/oder die dem ersten und/oder zweiten Schalter zugeordneten Signalcodiermittel zumindest eine Zenerdiode aufweisen, um den zumindest dritten Betriebszustand in von dem ersten und/oder zweiten Betriebszustand unabhängiger Weise zu realisieren. Mit einer derartigen Zenerdiode kann bspw. eine Spannungsänderung bzw. -begrenzung des Steuersignals hervorgerufen werden.

Weitere Merkmale und Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine schematische Gesamtdarstellung einer elektro-chirurgischen Vorrichtung, die drei Betriebsarten ermöglicht;
- Fig. 2: eine Schaltskizze einer bekannten Vorrichtung mit zwei Schaltern zum Umschalten zwischen zwei Betriebsarten;
- Fig. 3: eine Schaltskizze der Vorrichtung in Fig. 1, bei der die Vorrichtung in Fig. 2 mit zwei Schaltern auf drei Schalter erweitert wurde; und
- Fig. 4: eine Schaltskizze eines der Erfindung Ausführungsbeispiels zur Verwendung in der Vorrichtung in Fig. 1.

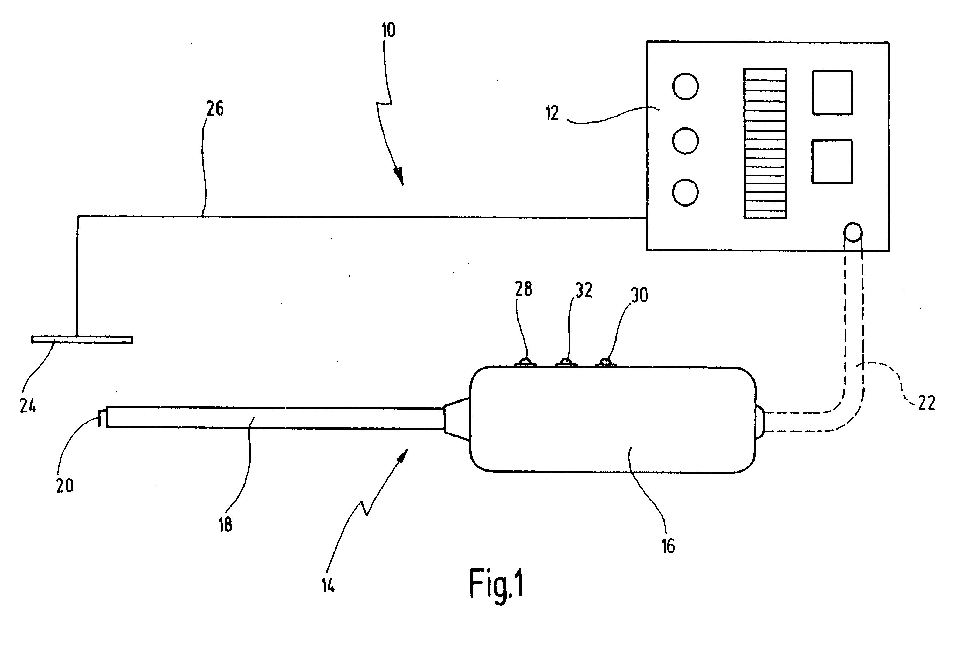

In Fig. 1 ist eine mit dem allgemeinen Bezugszeichen 10 versehene elektrochirurgische Vorrichtung schematisch dargestellt. Die Vorrichtung 10 wird im Rahmen der HF-Chirurgie verwendet.

Die Vorrichtung 10 weist einen HF-Generator 12 auf, der hochfrequente Ströme bzw. Spannungen von einigen hundert kHz erzeugt. Der HF-Generator 12 ist in der Lage, Hochfrequenzströme mit einer Ausgangsleistung, die zum Koagulieren von Gewebe geeignet ist, sowie Hochfrequenzströme mit einer Ausgangsleistung zu erzeugen, die zum Schneiden von Gewebe geeignet ist.

Die Vorrichtung 10 weist weiterhin ein HF-Instrument 14 auf, mit dem die vom HF-Generator 12 erzeugten Hochfrequenzströme im menschlichen oder tierischen Körper auf das zu behandelnde Gewebe appliziert werden können.

Das HF-Instrument 14 weist an seinem proximalen Ende ein Handstück 16 sowie einen mit dem Handstück 16 verbundenen Schaft 18 auf, der langerstreckt und im Durchmesser klein bauend ausgebildet ist, so daß er sich für die minimal-invasive Chirurgie eignet.

Am distalen Ende des Schafts 18 ist eine Elektrode 20 angeordnet, die als aktive Elektrode mit dem hochfrequenten Strom des HF-Generators 12 beaufschlagbar ist. Der HF-Generator 12 ist dazu über ein entsprechendes Kabel 22 mit dem Handstück 16 des HF-Instruments 14 verbunden, und über eine entsprechende nicht dargestellte Stromzuführung wird der hochfrequente Strom des HF-Generators durch das Handstück 16 und durch den Schaft 18 zu der aktiven Elektrode 20 geführt.

Mit dem HF-Generator 12 ist des weiteren eine Neutralelektrode 24 über eine Leitung 26 verbunden, wobei die Neutralelektrode 24 üblicherweise mit der Körperoberfläche des Patienten verbunden wird, um den HF-Stromkreis über die Körperoberfläche des Patienten zu schließen. Die in Fig. 1 dargestellte Anordnung ist demnach eine monopolare HF-Anordnung, wobei die vorliegende Erfindung jedoch nicht auf eine derartige monopolare Anwendung beschränkt ist, sondern auch für bipolare Anwendungen geeignet ist. Bei bipolaren Anwendungen ist die Neutralelektrode in unmittelbarer Nähe der aktiven Elektrode 20 am HF-Instrument selbst angeordnet.

Am Handstück 16 ist ein erster Schalter 28 in Form einer ersten Taste 28, ein zweiter Schalter 30 in Form einer zweiten Taste und ein dritter Schalter 32 in Form einer dritten Taste angeordnet.

Die drei Schalter 28, 30 und 32 dienen dazu, bei ihrer wahlweisen Betätigung einen bestimmten Betriebszustand des HF-Generators zu aktivieren.

Ohne Beschränkung der Allgemeinheit ist bei dem in Fig. 1 dargestellten Ausführungsbeispiel vorgesehen, daß bei Betätigung des ersten Schalters 28 ein Betriebszustand des HF-Generators 12 aktiviert wird, der der Betriebsart "Koagulieren" entspricht, d.h. der HF-Generator 12 erzeugt einen hochfrequenten Strom geringerer Ausgangsleistung und gegebenenfalls zeitlich unterbrochen. Bei Betätigung des zweiten Schalters 30 wird ein zweiter Betriebszustand des HF-Generators 12 aktiviert, der der Betriebsart "Schneiden" entspricht, d.h. in diesem Betriebszustand erzeugt der HF-Generator einen Hochfrequenzstrom mit einer gegenüber dem ersten Betriebszustand höheren Ausgangsleistung und ohne zeitliche Unterbrechung. Bei Betätigung des dritten Schalters 32 wird ein dritter Betriebszustand des HF-Generators 12 aktiviert, der einer dritten Betriebsart "schnelles, abwechselndes Aktivieren des Schneide- und Koagulationsstromes" entspricht, d.h. in diesem Betriebszustand erzeugt der HF-Generator abwechselnd Hochfrequenzströme, die zum Koagulieren bzw. zum Schneiden geeignet sind.

Die Schalter 28, 30 und 32 sind vorzugsweise in Form von Tasten ausgebildet, d.h. eine Aktivierung des entsprechenden Betriebszustandes erfolgt jeweils so lange, wie die entsprechende Taste gedrückt gehalten wird.

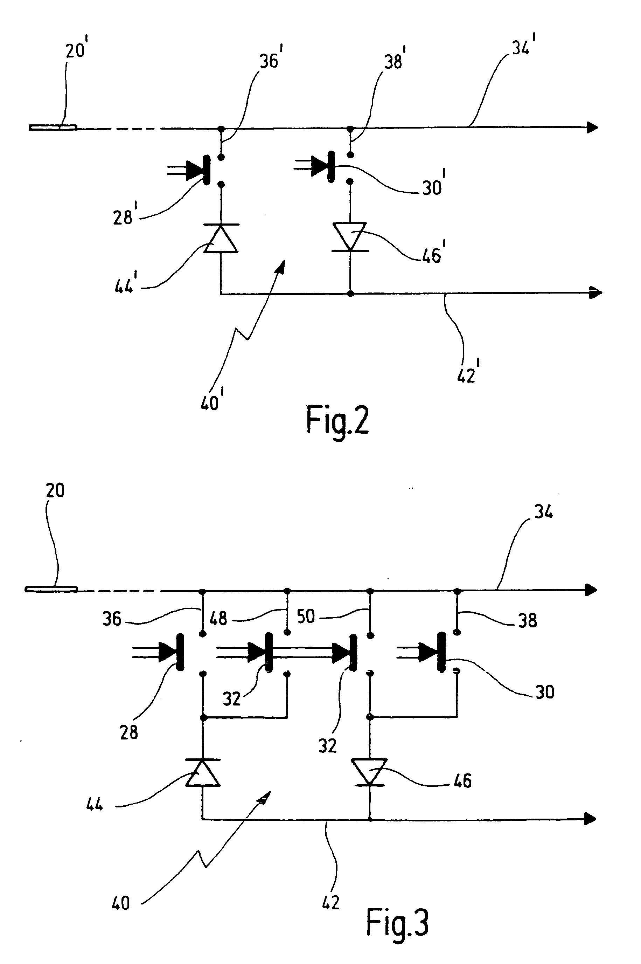

Mit Bezug auf Fig. 2 und 3 wird nun im folgenden anhand zweier Schaltbilder die Aktivierung der Betriebszustände des HF-Generators 12 näher beschrieben.

In Fig. 2 ist zunächst eine Schaltskizze eines aus dem Stand der Technik bekannten HF-Instruments dargestellt, das nur zwei Schalter 28' und 30' aufweist, um zwischen den Betriebsarten "Koagulieren" und "Schneiden" umzuschalten. Mit dem HF-Instrument 14 in Fig. 1 vergleichbare Komponenten wurden mit den gleichen Bezugszeichen, ergänzt durch einen hochgestellten Strich, versehen. Die Elektrode 20' ist über eine Hauptleitung 34', die sich durch den Schaft und das Handstück des HF-Instruments hindurch erstreckt, mit dem HF-Generator verbunden.

Von der Hauptleitung 34' gehen Zweigleitungen 36' und 38' ab, über die die Schalter 28' und 30' mit der Hauptleitung 34' verbunden sind.

Den Schaltern 28' und 30' sind Signalcodiermittel 40' zugeordnet.

Die Schalter 28' und 30' sind ferner über die Zweigleitungen 36' und 38' mit einer im gezeigten bevorzugten Ausführungsbeispiel gemeinsamen Steuersignalleitung 42' verbunden, die wiederum mit dem HF-Generator 12' verbunden ist.

Neben dem Hochfrequenzstrom, mit dem die Elektrode 20' beaufschlagt wird, wird über die Hauptleitung 34' ein Steuereingangssignal eingespeist, aus dem die Signalcodiermittel 40' in Abhängigkeit des Schaltzustandes des ersten Schalters 28' bzw. des zweiten Schalters 30' ein entsprechendes Steuersignal erzeugen, das über die Steuersignalleitung 42' wieder in den HF-Generator 12' zurückgeführt wird, um den Betriebszustand zu aktivieren, der dem entsprechenden Schalter 28' oder 30' zugeordnet ist.

Die Signalcodiermittel 40' weisen eine erste Diode 44' und eine zweite Diode 46' auf, die zueinander gegenpolig mit der Steuersignalleitung 42' verbunden sind.

Die Funktionsweise der in Fig. 2 dargestellten Schaltung wird nun näher beschrieben. Als Steuereingangssignal wird in die Hauptleitung 34' ein vorzugsweise niederfrequenter Wechselstrom zusätzlich zu dem hochfrequenten Wechselstrom eingespeist, mit dem die Elektrode 20' beaufschlagt wird.

Wird nun der erste Schalter 28' betätigt, d.h. geschlossen, schließt sich der Stromkreis für diesen zusätzlich eingespeisten Wechselstrom über die Zweigleitung 36', den ersten Schalter 28', die erste Diode 44' und die Steuersignalleitung 42'. Die erste Diode 44' läßt dabei jeweils nur die positive Halbwelle des Wechselstroms hindurch. Die positive Halbwelle des zusätzlich eingespeisten Wechselstroms dient nun als Steuerausgangssignal, das über die Steuersignalleitung 42' dem HF-Generator 12 zugeführt wird, um den ersten Betriebszustand zu aktivieren, d.h. um die Betriebsart "Koagulieren" zu aktivieren.

Wird anstelle des ersten Schalters 28' der zweite Schalter 30' betätigt, d.h. geschlossen, schließt sich der Stromkreis für den zusätzlich eingespeisten Wechselstrom über die Zweigleitung 38', den zweiten Schalter 30', die zweite Diode 46' und die gemeinsame Steuersignalleitung 42'. Die zweite Diode 46' läßt umgekehrt zur Diode 44' nur die negative Halbwelle des zusätzlich eingespeisten Wechselstroms hindurch, wobei diese negative Halbwelle nun als Steuerausgangssignal über die gemeinsame Steuersignalleitung 42' dem HF-Generator 12' zugespeist wird, wodurch entsprechend der zweite Betriebszustand des HF-Generators 14' aktiviert wird. Dadurch wird nun die Betriebsart "Schneiden" aktiviert.

In Fig. 3 ist nun die entsprechende Schaltskizze für die Vorrichtung in Fig. 1 dargestellt, in der sich alle Schaltelemente aus Fig. 2 wiederfinden, wodurch die konstruktive Einfachheit der vorliegenden Erfindung deutlich wird. Fig.3 ist allerdings kein Ausführungsbeispiel der Erfindung, sondern dient dem leichteien Verständnis der Erfindung.

Ohne eine weitere Steuersignalleitung zu erfordern, ist nämlich der dritte Schalter 32 ebenfalls mit der Steuersignalleitung 42 verbunden. Die Signalcodiermittel 40 sind so ausgebildet, daß bei Betätigung des dritten Schalters 32, d.h. wenn der dritte Schalter 32 geschlossen wird, ein weiteres Steuerausgangssignal zum Aktivieren des dritten Betriebszustandes des HF-Generators 12 erzeugt wird, wobei dieses erzeugte Steuerausgangssignal über die mit den Schaltern 28 und 30 gemeinsame Steuersignalleitung 42 dem HF-Generator 12 zugespeist wird.

In dem gezeigten Ausführungsbeispiel ist dies dadurch realisiert, daß der dritte Schalter 32 mit dem ersten Schalter 28 und dem zweiten Schalter 30 derart verbunden ist, daß sein Schließzustand einem gleichzeitigen Schließzustand des ersten Schalters 28 und des zweiten Schalters 30 entspricht. Dazu ist der dritte Schalter 32 über zwei weitere Zweigleitungen 48 und 50 einerseits mit der Hauptleitung 34 und andererseits mit den Zweigleitungen 36 und 38 des ersten Schalters 28 bzw. des zweiten Schalters 30 verbunden.

Beim Schließen des dritten Schalters 32 wird nun der Stromkreis sowohl über die erste Diode 44 als auch über die zweite Diode 46 geschlossen, wodurch der zusätzlich in die Hauptleitung 34 eingespeiste Wechselstrom unverändert über die gemeinsame Signalleitung 42 wieder dem HF-Generator 12 zugeführt wird, wodurch der dritte Betriebszustand des HF-Generators 12 aktiviert wird, der im vorliegenden Ausführungsbeispiel darin besteht, daß zwischen den Betriebsarten "Koagulieren" und "Schneiden" abwechselnd hin- und hergeschaltet wird. Für den dritten Schalter 32 und damit für den dritten Betriebszustand wird daher weder eine zusätzliche Steuersignalleitung noch ein zusätzliches in die Hauptleitung 34 eingespeistes Steuereingangssignal benötigt, noch wird bei dem HF-Generator 12 eine zusätzliche Signalerkennung benötigt. Das schnelle abwechselnde Aktivieren des Schneide- und Koagulationsstromes beim Betätigen des dritten Schalters 32 erfolgt nämlich mit der Frequenz des als Steuereingangssignal zusätzlich eingespeisten Wechselstromes, wenn der dritte Schalter 32 geschlossen ist und dementsprechend die positive und die negative Halbwelle des zusätzlich eingespeisten Wechselstromes über die Steuersignalleitung 42 dem HF-Generator 12 wieder zugeführt wird.

Die Frequenz des eingespeisten Wechselstromes kann dabei vorteilhafterweise am HF-Generator voreinstellbar sein.

Wie aus Fig. 3 hervorgeht, ist der dritte Schalter 32 parallel zu dem ersten Schalter 28 und parallel zu dem zweiten Schalter 30 geschaltet.

Anstelle einer elektrischen Parallelschaltung des dritten Schalters 32 mit den Schaltern 28 und 30 kann die gleiche Wirkung im Rahmen der vorliegenden Erfindung dadurch erzielt werden, daß der dritte Schalter 32 mit dem ersten und dem zweiten Schalter 28 und 30 mechanisch gekoppelt ist, derart, daß bei Betätigen des dritten Schalters 32 der erste und der zweite Schalter 28 und 30 gleichzeitig geschlossen werden. Die mechanische Kopplung muß dabei jedoch so ausgestaltet sein, daß sich der erste Schalter 28 und der zweite Schalter 30 auch weiterhin unabhängig voneinander betätigen lassen, um den ersten Betriebszustand und den zweiten Betriebszustand wahlweise aktivieren zu können.

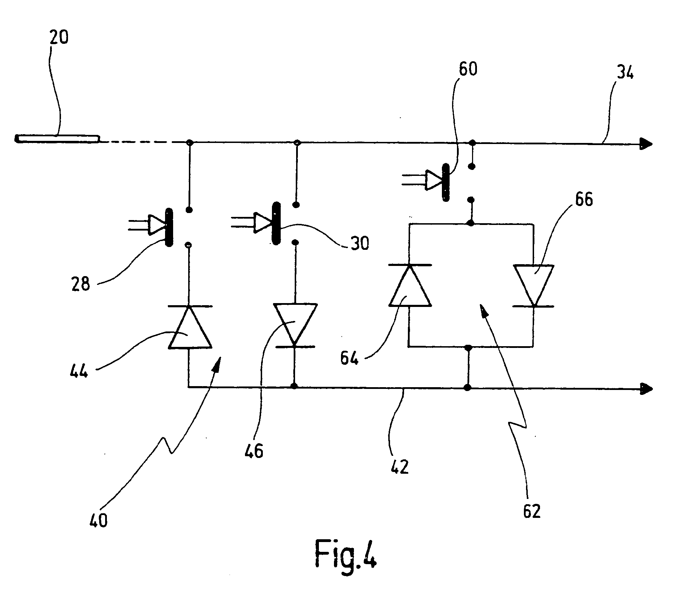

In Fig. 4 ist ein erfindungsgemäßes Ausführungsbeispiel einer Schaltung mit zumindest einem dritten Schalter 60 dargestellt. Gleiche Teile wie in Fig. 3 wurden mit dem gleichen Bezugszeichen versehen.

Anstatt daß dem dritten Schalter 60 dieselben Signalcodiermittel 40 wie dem ersten Schalter 28 und dem zweiten Schalter 30 zugeordnet sind, sind dem dritten Schalter 60 eigene Signalcodiermittel 62 zugeordnet. Die Signalcodiermittel 62 weisen eine Parallelschaltung aus einer ersten Diode 64 und einer zweiten Diode 66 auf, die zueinander gegenpolig parallel geschaltet sind. Die Diode 64 entspricht dabei der Diode 44 und die Diode 66 der Diode 46.

Bei Schließen des Schalters 60 erfolgt der Stromfluß von der Hauptleitung 34 kommend gleichzeitig über die Dioden 64 und 66, wodurch der gleiche Schaltzustand erreicht wird, wie wenn bei der Schaltung gemäß Fig. 3 der Schalter 32 geschlossen wird. Jedoch wird mit der Schaltung gemäß Fig. 4 ggf. eine bessere "Gleichzeitigkeit" erreicht, da mechanisch nur der Schalter 60 geschlossen wird, der entsprechend nicht als Doppelschalter ausgebildet ist.

Der durch den dritten Schalter 60 realisierte dritte Betriebszustand des HF-Generators 12 ist der gleiche, der auch bei Schließen des Schalters 32 eingestellt wird.

Anstelle wie zuvor beschrieben dem dritten Schalter eine erste Diode 64 und eine zweite Diode 66 zuzuordnen, die den Dioden 44 und 46 entsprechen, die dem ersten Schalter 28 und dem zweiten Schalter 30 zugeordnet sind, können die Dioden 64 und 66 auch durch solche Dioden ersetzt werden, die gegenüber den Dioden 44 und 46 andere Codiereigenschaften aufweisen. Bspw. könnten, wie dies in einer bevorzugten Ausgestaltung vorgesehen ist, die Dioden 64 und 66 durch zumindest eine Zenerdiode ersetzt werden, die eine Spannungsbegrenzung des Steuersignals bewirken. Hierdurch kann ein vollständig neuer, dritter Betriebszustand am HF-Generator aktiviert werden.

Im übrigen könnten auch umgekehrt die Dioden 44 und 46 durch Zenerdioden ersetzt sein.

## Patentansprüche

1. Elektrochirurgische Vorrichtung, mit einem HF-Generator (12) und mit einem HF-Instrument (14), wobei das HF-Instrument (14) einen ersten Schalter (28) und einen zweiten Schalter (30) aufweist, dem ersten Schalter (28) ein erster Betriebszustand und dem zweiten Schalter (30) ein zweiter Betriebszustand des HF-Generators (12) zugeordnet ist, ferner der erste Schalter (28) und der zweite Schalter (30) mit zumindest einer Steuersignalleitung (42) verbunden sind, wobei weiterhin dem ersten Schalter (28) und dem zweiten Schalter (30) Signalcodiermittel (40) zugeordnet sind, die in Abhängigkeit des Schaltzustandes des ersten und des zweiten Schalters (28, 30) aus einem Steuereingangssignal unterschiedliche Steuerausgangssignale zum wahlweisen Aktivieren des ersten Betriebszustandes oder des zweiten Betriebszustandes des HF-Generators (12) erzeugen, die dem HF-Generator (12) über die gemeinsame Steuerleitung zugespeist werden, wobei das HF-Instrument (14) weiterhin zumindest einen dritten Schalter (60) aufweist, dem zumindest ein dritter Betriebszustand des HF-Generators (12) zugeordnet ist, wobei der dritte Schalter (60) mit der zumindest einen Steuersignalleitung (42) derart verbunden ist, daß bei Betätigung des dritten Schalters (60) ein weiteres Steuerausgangssignal zum Aktivieren des dritten Betriebszustandes erzeugt wird, das über die zumindest eine Steuersignalleitung (42) dem HF-Generator (12) zugespeist wird, **dadurch gekennzeichnet, daß** dem dritten Schalter (60) eigene Signalcodiermittel (62) zugeordnet sind.

2. Vorrichtung nach Anspruch 1, **dadurch gekennzeichnet, daß** die dem dritten Schalter (60) zugeordneten Signalcodiermittel (62) einer Parallelschaltung der dem ersten und zweiten Schalter (28, 30) zugeordneten Signalcodiermittel (40) entsprechen.

3. Vorrichtung nach Anspruch 1, **dadurch gekennzeichnet, daß** die dem dritten Schalter (60) zugeordneten Steuercodiermittel (62) andere Codiereigenschaften aufweisen als die dem ersten und/oder zweiten Schalter (28, 30) zugeordneten Signalcodiermittel (40).

4. Vorrichtung nach einem der Ansprüche 1 bis 3, **dadurch gekennzeichnet, daß** die Signalcodiermittel (62) eine dem ersten Schalter (28) zugeordnete erste Diode (44) und eine dem zweiten Schalter (30) zugeordnete zweite Diode (46) aufweisen, wobei die erste und zweite Diode (44, 46) gegenpolig mit der Steuersignalleitung verbunden sind.

5. Vorrichtung nach Anspruch 3 oder 4, **dadurch gekennzeichnet, daß** die dem dritten Schalter (60) zugeordneten Signalcodiermittel (62) und/oder die dem ersten und/oder zweiten Schalter (28, 30) zugeordneten Signalcodiermittel (40) zumindest eine Zenerdiode aufweisen.

## Claims

1. An electrosurgical apparatus, comprising an HF generator (12) and an HF instrument (14), the HF instrument (14) having a first switch (28) and a second switch (30), the first switch (28) being assigned a first operating state, and the second switch (30) being assigned a second operating state of the HF generator (12), the first switch (28) and the second switch (30) further being connected to at least one control signal line (42), there being assigned, furthermore, to the first switch (28) and the second switch (30) signal coding means (40) that, as a function of the switching state of the first and the second switch (28, 30), generate from a control input signal different control output signals for the optional activation of the first operating state or of the second operating state of the HF generator (12), which are fed to the HF generator (12) via the common control line, wherein the HF instrument (14) furthermore has at least a third switch (32; 60), which is assigned at least a third operating state of the HF generator (12), the third switch (32; 60) being connected to the at least one control signal line (42) in such a way that, upon actuation of the third switch (32; 60), a further control output signal is generated for the activation of the third operating state and is fed to the HF generator (12) via the at least one control signal line (42), **characterized in that** signal coding means (62) of their own are assigned to the third switch (60).

2. The apparatus of claim 1, **characterized in that** the signal coding means (62) assigned to the third switch (60) correspond to a parallel connection of the signal coding means (40) assigned to the first and second switches (28, 30).

3. The apparatus of claim 1, **characterized in that** the signal coding means (62) assigned to the third switch (60) have other coding properties than the signal coding means (40) assigned to the first and/or second switches (28, 30).

4. The apparatus of anyone of claims 1 through 3, **characterized in that** the signal coding means (40; 62) have a first diode (44), assigned to the first switch (28), and a second diode (46), assigned to the second switch (30), the first and second diodes (44, 46) being connected to the control signal line with reversed polarity.

5. The apparatus of claim 3 or 4, **characterized in that** the signal coding means (62) assigned to the third switch (60) and/or the signal coding means (40) assigned to the first and/or second switches (28, 30) have at least one Zener diode.

## Revendications

1. Dispositif électrochirurgical, comportant un générateur haute fréquence (12) et un instrument haute fréquence (14), l'instrument haute fréquence (14) étant muni d'un premier interrupteur (28) et d'un deuxième interrupteur (30), le premier interrupteur (28) étant associé à un premier mode de service du générateur haute fréquence (12) et le deuxième interrupteur (30) étant associé à un deuxième mode de service de celui-ci, en outre, le premier interrupteur (28) et le deuxième interrupteur (30) sont reliés à au moins une ligne de transmission de signaux de commande (42), le premier (28) et le deuxième interrupteur (30) étant associés, par ailleurs, à des moyens de codage des signaux (40) qui, en fonction de la position du premier et du deuxième interrupteur (28, 30), génèrent à partir d'un signal d'entrée différents signaux de sortie commandant au choix l'activation du premier mode de service ou du deuxième mode de service du générateur haute fréquence (12), lesquels signaux sont acheminés, via la ligne de commande commune, dans le générateur haute fréquence (12), l'instrument haute fréquence (14) étant muni d'au moins un troisième interrupteur (60), qui est associé au moins à un troisième mode de service du générateur haute fréquence (12), le troisième interrupteur (60) étant relié à ladite au moins une ligne de transmission de signaux de commande (42), de telle sorte que, lors de l'activation du troisième interrupteur (60), un autre signal de sortie est généré pour commander l'activation du troisième mode de service, lequel signal est acheminé, via ladite au moins une ligne de transmission de signaux de commande (42), dans le générateur haute fréquence (12), **caractérisé en ce que** des moyens de codage des signaux (62) qui lui sont propres sont associés au troisième interrupteur (60).

2. Dispositif selon la revendication 1, **caractérisé en ce que** les moyens de codage des signaux (62) associés au troisième interrupteur (60) correspondent à un montage en parallèle des moyens de codage des signaux (40) associés au premier et au deuxième interrupteur (28, 30).

3. Dispositif selon la revendication 1, **caractérisé en ce que** les moyens de codage des signaux (62) associés au troisième interrupteur (60) présentent d'autres propriétés de codage que les moyens de codage des signaux (40) associés au premier et/ou au deuxième interrupteur (28, 30).

4. Dispositif selon l'une des revendications 1 à 3, **caractérisé en ce que** les moyens de codage des signaux (62) comportent une première diode (44), associée au premier interrupteur (28), et une deuxième diode (46), associé au deuxième interrupteur (30), la première et la deuxième diode (44, 46) étant reliées en opposition de pôle avec la ligne de transmission des signaux de commande.

5. Dispositif selon la revendication 3 ou 4, **caractérisé en ce que** les moyens de codage des signaux (62) associés au troisième interrupteur (60) et/ou les moyens de codage des signaux (40), associés au premier et/ou deuxième interrupteur (28, 30), comportent au moins une diode de Zener.
